# EUROPEAN PATENT SPECIFICATION

(11) **EP 3 189 848 B1**
(45) Date of publication and mention of the grant of the patent: **27.02.2019**
(21) Application number: 15801220.3
(22) Date of filing: 01.09.2015
(51) Int. Cl.: A61K 38/06, A61K 31/7008, A61K 31/737, A61P 19/02

(54) **A COMPOSITION COMPRISING TRIPEPTIDES FOR THE TREATMENT OF DISEASES OF THE JOINTS AND THE SPINE**
ZUSAMMENSETZUNG ENTHALTEND TRIPEPTIDE ZUR VORBEUGUNG UND BEHANDLUNG VON ERKRANKUNGEN DER GELENKE UND DER WIRBELSÄULE
COMPOSITIONS COMPRENANT DES TRIPEPTIDES POUR LA PRÉVENTION ET LE TRAITEMENT DE MALADIES DES ARTICULATIONS ET DE LA COLONNE VERTÉBRALE

(30) Priority: 05.09.2014 EA 201400887
(43) Date of publication of application: 12.07.2017
(73) Proprietor: Zamerton Holdings Limited, 6018 Larnaka (CY)
(72) Inventor: BELOV, Evgeniy Yurievich, Obninsk Kaluzhskaya obl. 249032 (RU); CHERTORIZHSKIY, Evgeniy Aleksandrovich, Obninsk Kaluzhskaya obl. 249031 (RU); BELY, Petr Aleksandrovich, Moscow 127055 (RU)
(74) Representative: Icosa
(86) International application number: PCT/IB2015/001830
(87) International publication number: WO 2016/034941

(56) References cited:
- WO-A1-2007/139433
- WO-A2-2014/106772
- US-A1- 2002 068 718
- MAEHASHI K ET AL: "ISOLATION OF PEPTIDES FROM AN ENZYMATIC HYDROLYSATE OF FOOD PROTEINS AND CHARACTERIZATION OF THEIR TASTE PROPERTIES", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 63, no. 3, 1 January 1999 (1999-01-01), pages 555-559, XP009050532, ISSN: 0916-8451, DOI: 10.1271/BBB.63.555

## Description

The invention relates to medicine and concerns a pharmaceutical composition comprising peptides Lys-Asp-Glu, Ala-Asp-Glu, Asp-Glu-Gly and its application for prevention or treatment of diseases of the joints and the spine. The invention also relates to a composition intended for prevention and treatment of diseases of the joints comprising a mixture of peptides Lys-Asp-Glu, Ala-Asp-Glu, Asp-Glu-Gly in combination with glucosamine or its pharmaceutically acceptable salts and/or chondroitin or its pharmaceutically acceptable salts. Diseases of the joints may include arthritis, osteoarthritis, and osteochondrosis of the spine, as well as other conditions and diseases accompanied by joint lesions and pain.

### Prior art

The causes of the joint diseases are extremely diverse and still largely unknown. Only the causes of infectious specific arthritises (tuberculous, gonorrheal, brucellar, etc.) attributed to specific infections have been well understood. As for the remaining diverse forms of joint lesions, then, according to modern concepts, their development is associated with a complex of various internal and external factors.

Nevertheless, the final stages of these diseases are associated with lesions and further destruction of cartilaginous tissue, which is necessary for normal functioning of joints.

For instance, such joint disease as osteoarthritis represents a heterogeneous group of diseases of various etiologies, but with similar biological, morphological, and clinical manifestations and outcomes, which result from damage caused to all joint components, primarily articular cartilages as well as subchondral portions of bones, synovial membranes, ligaments, capsules, and periarticular muscles. As a chronic degenerative-dystrophic disease of the joints, osteoarthritis is characterized by progressive destruction of articular cartilages, proliferative reaction of cartilaginous and bone tissues, and is accompanied by reactive synovitis [Kovalenko V.N., Bortkiewich O.P. Osteoarthritis. A Practical Guide. 2nd ed., Kiev: Morion, 2005, p. 592].

Osteoarthritis is the most common joint disease, which affects at least 20% of the world population (it usually starts to develop after the age of 40), with almost 25% of the patients being unable to cope with basic activities of daily living. The progression of the disease, accompanied by persistent pain experienced in the affected joints, represents the cause of the disability of such patients, thereby exerting severe economic and psychological impacts not only on the patients but also on their families.

The development of osteoarthritis leads to significant economic, social, and psychological costs associated with widespread distribution, frequent disability of the patients, and high economic expenditures, including costs of treatment of the underlying diseases, and prevention and treatment of potential complications associated with pharmacotherapy.

The same applies to other joint diseases.

The development of new and effective means and methods of treatment and prevention of joint diseases is a pressing issue of contemporary pharmacology and clinical medicine, since such discoveries would not only lead to increased levels of social activity and improvement in quality of life of the patients but also to reduction of healthcare costs associated with their maintenance.

A drug known as glucosamine has been developed for management of joint diseases along with a method of their treatment that is based on administration of the specified preparation, which is widely used, for example, in the treatment of patients with primary and secondary osteoarthritis to reduce joint pain and slow down the progression of the disease. However, this method based on administration of glucosamine cannot be considered sufficiently effective as its effects are associated with only one of the mechanisms of protection against damage to cartilage, specifically stimulation of biosynthesis of aminoglycans [Zupanets I.A. Experimental justification of application of glucosamine and its derivatives in medicine: Dissertation in the form of a scientific paper of a Doctor of Medical Sciences. Kupavna, 1993, page 90].

Meanwhile, these diseases lead to degenerative-dystrophic joint lesions accompanied by progressive destruction of articular cartilages, proliferative reaction of cartilaginous and bone tissues and development of synovitis. In addition, comorbidities causing circulatory disorders (atherosclerosis, diabetes, etc.), which may contribute to damage caused to cartilaginous tissue and development of joint diseases, constitute one important trigger of their development.

Based on the foregoing, there is a need for new agents characterized by chondroprotective activity, analgesic, repairing, and vasoactive properties, and methods of treatment of joint diseases of various etiologies based on the application of the specified compounds, as the action of such agents will be directed at correcting primary manifestations of the diseases and their causes.

Prior art contains records of a pharmaceutical composition intended for prevention and treatment of the musculoskeletal system disorders, comprising peptide Ala-Glu-Asp, and a method of its application for management of the above-mentioned diseases. This peptide, constituting a part of a known composition, is characterized by pronounced chondroprotective activity with respect to bone and cartilaginous tissues. The dose of the peptide ranges from 0.01 to 100 mg/kg body weight (Russian Federation Patent 2299741).

Prior art contains records of peptide Lys-Glu-Asp (Russian Federation Patent No. 2295970) used for correction of the metabolic vascular syndrome and diseases accompanied by impaired vascular permeability and capillary fragility. The effective dose of the peptide ranges from 0.01 to 100 mg/kg body weight. This peptide has never been applied for treatment of joint diseases.

Peptide Lys-Glu (Russian Federation Patent No. 2139085) characterized by ability to stimulate repair processes has also been discovered. The effective dose of the peptide ranges from 0.01 to 100 mg/kg body weight. This peptide has also never been used for management of joint diseases. Application of a combination of chondroitin sulfate and glucosamine as an agent capable of stimulation of regeneration of cartilaginous tissue has also been previously claimed (Reference Book of the Register of Medicinal Products, 2009). Preparation "Teraflex" contains 500 mg of glucosamine hydrochloride and 400 mg of sodium chondroitin sulfate. Preparation "Kondronova capsules" contains 250 mg of glucosamine sulfate and 200 mg of chondroitin sulfate. Preparation "Kondronova ointment for external use" contains 25 mg of glucosamine sulfate and 50 mg of chondroitin sulfate. Preparation "Artra" contains 500 mg of glucosamine hydrochloride and 500 mg of sodium chondroitin sulfate.

The development of new, safe, and effective medicinal products based on peptides characterized by chondroprotective, analgesic, and other valuable properties is a pressing issue of contemporary pharmacology.

### Summary of the invention

A pharmaceutical composition intended for prevention and treatment of joint diseases comprising peptides Lys-Asp-Glu, Ala-Asp-Glu, Asp-Glu-Gly in effective amount within a pharmaceutically acceptable carrier is proposed. In the preferred embodiment of the invention, the weight ratio of peptides Lys-Asp-Glu, Ala-Asp-Glu, Asp-Glu-Gly within a pharmaceutically acceptable carrier must be in the following range: (0.01-1):(0.01-1):(0.01-1).

An agent for prevention and treatment of joint diseases, comprising a mixture of peptides Lys-Asp-Glu, Ala-Asp-Glu, Asp-Glu-Gly in combination with glucosamine or its pharmaceutically acceptable salts and/or chondroitin (chondroitin sulfate) or its pharmaceutically acceptable salts is also proposed, whose application enables one to achieve a synergistic effect, which augments the effectiveness of therapy of inflammatory diseases of the joints. The weight ratio of peptides Lys-Asp-Glu, Ala-Asp-Glu, Asp-Glu-Gly within the specified agent must be in the range of (0.01-1):(0.01-1):(0.01-1); the remaining components are represented by glucosamine or its pharmaceutically acceptable salts and/or chondroitin or its pharmaceutically acceptable salts. In the preferred embodiment of the invention, the agent may contain glucosamine hydrochloride, sodium, potassium, or calcium salt of glucosamine sulfate as the glucosamine salt. In the preferred embodiment of the invention, the salt of chondroitin sulfate within the agent is represented by its sodium, potassium or calcium salt.

Application of the proposed compositions and the agent is accompanied by a pronounced analgesic effect and results in positive changes in the dynamics of clinical and biochemical parameters, contributes to normalization of the morphostructure of joint tissues (including the cytoarchitectonics of cartilaginous tissue accompanied by decrease in the number of chondrocytes undergoing apoptosis, particularly its final stages), and exerts a favorable effect on the metabolic processes that occur in chondrocytes (activation of the synthetic processes and normalization of the biopolymer composition of cartilaginous matrix).

Another aspect of the invention relates to methods of prevention and treatment of joint diseases entailing administration of a composition comprising peptides Lys-Asp-Glu, Ala-Asp-Glu, Asp-Glu-Gly in effective amounts to patients; or an agent comprising peptides Lys-Asp-Glu, Ala-Asp-Glu, Asp-Glu-Gly and glucosamine or its pharmaceutically acceptable salts and/or chondroitin or its pharmaceutically acceptable salts. In the preferred embodiment of the invention, the weight ratio of the peptides must be in the range of (0.01-1):(0.01-1):(0.01-1).

### Detailed description of the invention

The present invention proposes a pharmaceutical composition comprising peptides Lys-Asp-Glu, Ala-Asp-Glu, Asp-Glu-Gly in effective amounts as active ingredients along with a pharmaceutically acceptable carrier, which is characterized by anti-inflammatory and chondroprotective effects with respect to joint diseases.

Another aspect of the invention relates to an agent comprising peptides Lys-Asp-Glu, Ala-Asp-Glu, Asp-Glu-Gly as active ingredients in combination with glucosamine or its pharmaceutically acceptable salts and/or chondroitin or its pharmaceutically acceptable salts. In the preferred embodiment of the invention, the weight ratio of the peptides must be in the range of (0.01-1):(0.01-1):(0.01-1). In the preferred embodiment of the invention, the weight ratio of chondroitin or its salts and/or a glucosamine or its salts within the specified agent must be in the range of (1-9):(1-9).

In particular, the invention relates to an agent intended for prevention and treatment of joint diseases, comprising a pharmaceutical composition that contains the peptides and chondroitin or its salts with the following ratio of the components in weight percent:

| | |
|---|---|
| pharmaceutical composition comprising peptides Lys-Asp-Glu, Ala-Asp-Glu, Asp-Glu-Gly | - 0.01-1.00; |
| chondroitin or its salts | - remaining weight |

Another variant of the invention relates to an agent for prevention and treatment of joint diseases, comprising a pharmaceutical composition that contains the peptides and glucosamine or its salts with the following ratio of the components in weight percent:

| | |
|---|---|
| pharmaceutical composition comprising peptides Lys-Asp-Glu, Ala-Asp-Glu, Asp-Glu-Gly | - 0.01-1.00; |
| glucosamine or its salts | - remaining weight |

Another variant of the invention relates to an agent for prevention and treatment of joint diseases, comprising a pharmaceutical composition that contains the peptides, a mixture of chondroitin and/or its salts and glucosamine and/or its salts with the following ratio of the components in weight percent:

| | |
|---|---|
| pharmaceutical composition comprising peptides Lys-Asp-Glu, Ala-Asp-Glu, Asp-Glu-Gly | - 0,01-1,00; |
| a mixture of chondroitin or its salts and/or glucosamine or its salts | - remaining weight. |

The composition comprising peptides Lys-Asp-Glu, Ala-Asp-Glu, Asp-Glu-Gly in combination with chondroitin or its salts and/or glucosamine or its salts is characterized by a pronounced synergistic effect.

The chondroprotective and anti-inflammatory effects of peptides Lys-Asp-Glu, Ala-Asp-Glu, Asp-Glu-Gly, as well as peptides Lys-Asp-Glu, Ala-Asp-Glu, Asp-Glu-Gly in combination with chondroitin sulfate and/or glucosamine sulfate according to the invention was investigated on animal models of systemic arthritis and in patients suffering from diseases of the joints and the spine (Examples 2 and 3).

Other aspects of the invention relate to methods of prevention and treatment of disorders associated with diseases of the joints or the spine entailing administration of the pharmaceutical composition or the agent according to the invention to patients.

When the proposed methods of prevention and treatment of disorders associated with diseases of the joints and the spine are used, regimens and administered doses of the preparations depend on the stage of the relevant disease and the patient's condition. The daily dose depends on the severity of the disease, the patient's weight, and the desired effect. The dose is determined individually depending on the tolerability and clinical efficacy. In the most preferred embodiment of the invention, the daily dose of a mixture of peptides Lys-Asp-Glu, Ala-Asp-Glu, Asp-Glu-Gly ranges from 150 to 300 µg.

When the influence of the composition comprising peptides Lys-Asp-Glu, Ala-Asp-Glu, Asp-Glu-Gly on the intensity of apoptosis of the chondrocytes in the articular cartilages of rats with developing systemic steroid arthritis was investigated, it was discovered that the composition according to the invention was characterized by chondroprotective activity. The results of an electron microscopic investigation of the structure of the articular cartilages revealed decreased number of chondrocytes undergoing apoptosis (chondroptosis), especially its final stages, which was indicative of the fact that the composition according to the invention was characterized by an anti-apoptotic effect with respect to the chondrocytes.

Investigations of the level of the chondroprotective activity of the composition comprising peptides Lys-Asp-Glu, Ala-Asp-Glu, Asp-Glu-Gly under the conditions of development of experimental arthritis in animal models and in patients suffering from diseases of the joints and the spine demonstrated superior efficacy of the mixture of the peptides in comparison to glucosamine sulfate or chondroitin sulfate.

Furthermore, the chondroprotective and anti-inflammatory effects of a composition comprising peptides Lys-Asp-Glu, Ala-Asp-Glu, Asp-Glu-Gly and an agent comprising the composition of the peptides in combination with chondroitin sulfate and/or glucosamine sulfate are superior to those associated with glucosamine sulfate or chondroitin sulfate administered separately, which enables one to draw a conclusion regarding a synergistic effect attributed to the combined application. Meanwhile, increase in the amount of peptides within the agent is associated with increased efficacy of the specified agent accompanied by a pronounced analgesic effect.

The findings of the studies of the chondroprotective and anti-inflammatory effects of the peptide composition enables the application of the proposed composition as an effective therapeutic and preventive agent in the treatment of osteoarthritis of the spine and the peripheral joints, including osteoarthritises, arthropathies, and intervertebral osteoarthritis.

The peptides are obtained by fine chemical synthesis using conventional methods.

The pharmaceutical composition may be manufactured in the form of a tablet, nasal spray (dosated), sublingual aerosol (dosated), capsule, powder, solution, lyophilisate for preparation of solution, cream, ointment, liniment, gel, or drops for oral administration. Meanwhile, the tablets may be sublingual, sustained-release, and film-coated, and the solution may be for intravenous and intramuscular administration.

The invention is illustrated by the following examples.

Example 1. A study of the toxicity of a composition comprising peptides Lys-Asp-Glu, Ala-Asp-Glu, Asp-Glu-Gly.

The acute toxicity of the composition comprising peptides Lys-Asp-Glu, Ala-Asp-Glu, Asp-Glu-Gly was investigated using mice weighing 22-27 g at 24 hrs following intraperitoneal administration of the composition of the peptides at a dose of 2 mg/kg. Neither animal death nor significant toxic reactions were observed at 24 hrs following a single intraperitoneal administration of the peptide mixture. Hence, the composition comprising peptides Lys-Asp-Glu, Ala-Asp-Glu, Asp-Glu-Gly administered at a dose of 2 mg/kg (single intraperitoneal injection) does not induce any significant toxic reactions in mice.

It was demonstrated during this study that administration of the composition was not associated with induction of any negative reactions in the animals or the skin. Insignificant reversible hyperemic response was sometimes observed at the injection sites. No swelling, itching, irritation, or development of necrotic processes were observed.

Example 2. A study of the chondroprotective activity of a composition comprising peptides Lys-Asp-Glu, Ala-Asp-Glu, Asp-Glu-Gly, and an agent comprising peptides Lys-Asp-Glu, Ala-Asp-Glu, Asp-Glu-Gly and chondroitin sulfate and/or glucosamine sulfate using a rat-based model of systemic steroid arthritis.

Lyophilized powder consisting of peptides Lys-Asp-Glu, Ala-Asp-Glu, Asp-Glu-Gly was used to obtain the preparation for intramuscular injections.

The daily dose of the composition comprising peptides Lys-Asp-Glu, Ala-Asp-Glu, Asp-Glu-Gly was 50 µg/kg (at a weight ratio of the peptides of 1:1:1), 150 µg/kg (at a weight ratio of the peptides of 1:2:1), 300 µg/kg (at a weight ratio of the peptides of 0.1:1:10) or 600 µg/kg (at a weight ratio of the peptides of 1:1:3).

The daily dose of the composition comprising peptides Lys-Asp-Glu, Ala-Asp-Glu, Asp-Glu-Gly and glucosamine sulfate or chondroitin sulfate was 150 µg/kg of the peptides (at a weight ratio of the peptides of 1:2:1) and 50 mg/kg of glucosamine sulfate or chondroitin sulfate, or 300 µg/kg of the peptides (at a weight ratio of the peptides of 0.1:1:10) and 50 mg/kg of glucosamine sulfate or chondroitin sulfate, or 600 µg/kg of the peptides (at a weight ratio of the peptides of 1:1:3) and 50 mg/kg of glucosamine sulfate or chondroitin sulfate.

The daily dose of the composition comprising peptides Lys-Asp-Glu, Ala-Asp-Glu, Asp-Glu-Gly and glucosamine sulfate and chondroitin sulfate was 150 µg/kg of the peptides (at a weight ratio of the peptides of 1:2:1) and 50 mg/kg of glucosamine sulfate and chondroitin sulfate each, or 300 µg/kg of the peptides (at a weight ratio of the peptides of 0.1:1:10) and 50 mg/kg of glucosamine sulfate and chondroitin sulfate each.

Glucosamine sulfate and chondroitin sulfate at a daily dose of 50 mg/kg corresponding to the average effective dose with respect to anti-inflammatory activity was used as a control (produced by Protein Chemicals, Japan).

The study was conducted using white outbred male rats 4-5 months of age weighing 250-300 g obtained from the same litter (same age and weight, with normal primary clinical and biochemical analyses parameters). Modeling of steroid arthritis was achieved by three single intramuscular injections of dexamethasone administered at an interval of 1 week at a dose of 7 mg/kg. The first signs of the arthropathy were detected as early as week 2 of the experiment.

All animals following three intramuscular administrations of dexamethasone presented with clinical signs of the musculoskeletal system lesions. This was primarily manifested in the fact that the animals were lethargic and inactive. Swelling of the knee joints, decline in their range of motion, stiffness, and decreased stress tolerance were observed. In addition, decrease in appetite and deterioration of the fur coats were detected.

Starting from day 28 of the experiment, all animals received corresponding intramuscular doses of the preparations on a daily basis over 4 weeks. The degree of activity of the pathological process was evaluated using biochemical blood test parameters. The following biochemical parameters were used as the parameters for evaluation of the intensity of progression of the pathological process in the experimental groups: the contents of sialic acids, glycoproteins, and chondroitin sulfates in the serum, as these parameters reflect the intensity of progression of the degenerative-dystrophic processes in the connective tissue.

Clinical observation of the animals was implemented throughout the study; the functional state of the rat joints was monitored (degree of mobility, resistance to stress, swelling, hyperaemia). The animals were decapitated under ether anesthesia at the end of the study (on day 56) to harvest biomaterials for biochemical and histomorphological studies.

### I. Evaluation of the degree of activity of the pathological process through observing the dynamics of the contents of the primary connective tissue metabolites in the blood serum of rats with experimental arthritis

The parameters of the primary connective tissues metabolites, e.g. chondroitin sulfates (CS), glycoproteins (GPs), and sialic acids, represented the primary parameters for evaluation of the degree of chondroprotective activity within the framework of this study. Among the mentioned parameters, the most significant importance in the development of acute arthritis is assigned to the level of CS, as it represents the primary component of the articular cartilage matrix. The levels of GPs and sialic acids reflect mostly the acute phase pathological processes that occur in the connective tissue. Evaluation of the content of sialic acids in the blood serum was carried out using Hess's method [Kamyshnikov V.S. Clinical-biochemical laboratory diagnostics. Reference Book: volume 1, page 495; volume 2, page 463; 2nd edition, Minsk: Interpresservice, 2003]; the content of glycoproteins (GPs) in the blood serum - via a reaction with molybdenum sulfate reagent (Steinberg-Dotsenko method) [Medical laboratory technologies. Reference Book: volume 1, page 408; volume 2, page 600 / Editor A.I. Karpischenko. Intermedika, 2002]; and the content of chondroitin sulfate in the blood serum - using a method proposed by L.I. Slutsky [Slutsky L.I. Biochemistry of normal and pathologically altered connective tissue. Leningrad: Medicine, 1969, page 427]. Statistical processing of the results was performed using Student's t-test.

The findings of the study of the contents of the primary connective tissue metabolites in the blood serum of the rats are presented in Table 1.

**Table 1.**

| The dynamics of the contents of the primary connective tissue metabolites in the blood serum of the rats with experimental arthritis receiving the compositions according to the invention | | | | |
|---|---|---|---|---|
| Experimental conditions | Dose of the preparation | Chondroitin sulfate, g/l | Glycoprotei ns, g/l | Sialic acids, mmol/l |
| Day 28 | | | | |
| Control pathology (n=20) | - | 0.402±0.018 | 3.87±0.19 | 5.13±0.24 |

| Day 56 | | | | |
|---|---|---|---|---|
| Control pathology (n=20) | - | 0.426±0.020 | 4.15±0.22 | 4.83±0.18 |
| Composition of peptides | 50 µg/kg (1:1:1) n=10 | 0.366±0.029 | 3.79±0.30 | 4.26±0.18 |
| Lys-Asp-Glu, Ala-Asp-Glu, Asp-Glu-Gly | 150 µg/kg (1:2:1) n=15 | 0.310±0.026 | 3.18±0.25 | 4.07±0.2 |
| | 300 µg/kg (0.1:1:10) n=20 | 0.315±0.027 | 2.93±0.23 | 3.97±0.25 |
| | 600 µg/kg (1:1:3) n=21 | 0.319±0.028 | 3.12±0.21 | 4.00±0.25 |
| Glucosamine sulfate (n=12) | 50 mg/kg | 0.400±0.027 | 3.59±0.29 | 4.27±0.34 |
| Chondroitin sulfate (n=12) | 50 mg/kg | 0.382±0.025 | 3.97±0.3 | 4.51±0.32 |
| Composition (150 µg/kg) Glucosamine sulfate (50 mg/kg) (n=15) | | 0.304±0.01 | 2.5±0.12 | 3.2±0.15 |
| Composition (300 µg/kg) Chondroitin sulfate (50 mg/kg) (n=12) | | 0.302±0.013 | 2.35±0.15 | 3.28±0.1 |
| Composition (600 µg/kg) Chondroitin sulfate (50 mg/kg) (n=16) | | 0.305±0.02 | 2.24±0.11 | 3.15±0.13 |
| Composition (150 µg/kg) Glucosamine sulfate (50 mg/kg) Chondroitin sulfate (50 mg/kg) (n=20) | | 0.303±0.014 | 2.57±0.13 | 3.25±0.17 |
| Composition (300 µg/kg) Glucosamine sulfate (50 mg/kg) Chondroitin sulfate (50 mg/kg) (n=25) | | 0.301±0.015 | 2.45±0.12 | 3.18±0.14 |

| | | | | |
|---|---|---|---|---|
| Note: p< 0.05 | | | | |

As follows from the data presented in Table 1, administration of the composition according to the invention to the animals at doses of 50 µg/kg, 150 µg/kg, 300 µg/kg or 600 µg/kg was accompanied by significant reduction in the values of all investigated blood serum parameters in comparison to the control pathology group, which supports the fact that the composition according to the invention exerts a positive effect on cartilaginous tissue metabolism in rats with acute arthritis.

Moreover, in the case of administration of the peptide composition at doses of 150, 300 and 600 µg/kg, the reduction in the contents of CS, GPs and sialic acids in the blood serum was more significant than under the influence of the composition administered at a dose of 50 µg/kg, and the peptide composition administered at doses of 150, 300 and 600 µg/kg was superior to the known preparations containing glucosamine sulfate and chondroitin sulfate with respect to efficacy.

Application of the agent comprising peptides Lys-Asp-Glu, Ala-Asp-Glu, Asp-Glu-Gly in combination with glucosamine sulfate and/or chondroitin sulfate was accompanied by a synergistic effect with respect to increase in the level of chondroprotective activity; thus, the level of the investigated blood serum parameters was statistically significantly reduced to a greater extent in comparison to the level attained when the peptide composition was administered at doses of 150, 300, and 600 µg/kg.

Therefore, the studies of the contents of the primary connective tissue metabolites in the blood serum of the rats with experimental arthritis demonstrated the efficacy of the influence of the proposed composition of the peptides on metabolism impaired by administration of dexamethasone and support the fact that the composition is characterized by anti-inflammatory and chondroprotective activities. Meanwhile, administration of the composition in combination with glucosamine sulfate and chondroitin sulfate was accompanied by synergistic anti-inflammatory and chondroprotective effects. The dose interval between 150 and 300 µg/kg was determined to be most preferable.

Increase in the content of chondroitin-4-sulfate represents the most important biochemical parameter characterizing the intensity of the pathological changes occurring in the articular tissues of the animals suffering from systemic arthrosis, as the specified compound is the most abundant components of the articular cartilage matrix abandoning it as a result of degradation. Subsequently, aggravation of the pathological process progression is accompanied by increase in the content of keratan sulfates.

Significant reduction in both the fraction of hyaluronates and chondroitin-6-sulfate as well as the fraction of chondroitin-4-sulfate was observed during the study of the pharmacological activity of the composition according to the invention administered at a dose of 150 µg/kg, which is indicative of significant decrease in the activity of the pathological process in the cartilaginous tissue.

Meanwhile, the animals demonstrated intensified motor activity, increased exercise tolerance, and increased appetite; visual examination revealed normalization of the condition of the joints.

### II. A study of the influence of a mixture of the peptides on the articular cartilage structure of laboratory animals

In order to investigate the chondroprotective activity of the composition, a study of the condition of the morpho- and ultrastructure of the articular cartilages of the knee joints of the rats was carried out on day 56 of the experiment.

The histomorphological investigation of the articular cartilages was carried out using standard light microscopy techniques.

The morphometric measurements were carried out using an ocular micrometer: the cartilage thickness was determined using slide mounts; the measurements were taken at 3 individual points - the central part of the head, and the upper and lower edges. The thickness of the articular cartilages was expressed in conventional units. The density of chondrocytes per conventional unit area was also estimated. Statistical processing of the digital data obtained was performed using Student's t-test and non-parametric methods of analysis (Mann-Whitney U Test).

In order to conduct quantitative assessment of the condition of the major structures of the joints, the following assessment system was used:
Changes in the joints expressed in points were distributed as follows:
- 23-25 points - joint condition was deemed to be normal (smooth surface, cartilage thickness corresponded to thickness of normal articular cartilages, cartilage cell density was at the normal level - preservation of cytoarchitectonics, joint capsule and subchondral bone were unaffected);
- 19-22 points - mildly pronounced abnormalities in the structure of cartilage and joint capsule (weakly expressed pulping of the surface in certain areas, cartilage thickness reduction of up to 25% in certain areas, insignificant cell hyperplasia, mildly expressed signs of synovitis);
- 14-18 points - moderately pronounced changes in the structure of cartilage and joint capsule (reduced cell density in certain areas, mildly expressed signs of synovitis);
- 8-13 points - marked disruption of the structure of cartilage and joint capsule (pronounced signs of synovitis, expressed pulping of the surface exceeding 50% of the total area, absence of articular cartilage in certain areas, foci of lysis of bone trabeculae, expansion of intertrabecular spaces);
- 0-7 points - severe abnormalities, including destruction of articular surface, destruction of subchondral bone, hardening and fibrotization of joint capsule.

The study of the ultrastructure of the articular cartilages of the rats was performed using conventional electron microscopy techniques.

### Control pathology

Histological examination of the knee joints of the rats conducted on day 56 of the experiment revealed signs of pathology characterized by the development of hyperplastic, inflammatory, and destructive-dystrophic processes, which corresponds to the generally-accepted notion of pathomorphological features of the development of osteoarthritis. Meanwhile, not only the destruction of cartilaginous tissue was observed, but also the destruction of the soft tissue structures of the joints - synovial membranes, which was manifested in pronounced proliferation of the synoviocytes arranged in 6-7 or more rows resulting in a thickened and multicellular fold.

All animals in the control group were characterized by destructive-dystrophic lesions of the articular cartilages. The wearing out of the zonal structure of the cartilaginous tissue must be emphasized: layering was missing and the chondrocytes were arranged in random order. Dystrophic processes developed in the preserved cells: cells with both hypertrophic (containing an increased number of nucleoli) and pycnotic nuclei were encountered. The cytoplasm of the chondrocytes was vacuolated.

The reduced density and, consequently, the synthetic activity of the chondrocytes resulted in degenerative changes of the intercellular substance: the primary substance was characterized by uneven coloration, pulping of the matrix was observed, circumscribed cartilage resorption was encountered. The articular surfaces lost clarity of the boundaries.

The so-called bone excrescences - portions of the bone-derived interstitial tissue with enlarged vascular buds penetrating the cartilage and promoting enhanced calcification of the cartilaginous tissue - are characteristic of the cartilages of this animal group. This leads to thinning of the cartilage: in certain areas the median zone was represented by only 1-2 rows of cells.

Reduction in the thickness of the cartilages was the outcome of the development of the above described pathological processes. When quantitative assessment of the condition of the cartilages of the rats belonging to this group was carried out, the total score averaged 12.9, which was indicative of pronounced dystrophic abnormalities occurring in the structures of the cartilages and joint capsules.

### A mixture of the peptides at a dose of 50 µg/kg

When the condition of the morphostructure of the joints of the rats exposed to the composition according to the invention at a dose of 50 µg/kg was evaluated, the value of the cell density parameter increased to 18.2 conventional units and decrease in the number of joints with pathological manifestations was observed. Meanwhile, in contrast to the control pathology group, no areas of cartilage destruction and its replacement with connective tissue were discovered.

### A mixture of the peptides at a dose of 150 µg/kg

A study of the structure of the articular cartilages of the rats exposed to the composition at a dose of 150 µg/kg revealed significantly improved morphology. Average thickness of the articular cartilages was equal 16.5 conventional units, the value of the cell density parameter increased to 28.38 conventional units. The articular cartilages were characterized by flat surfaces with no visible signs of destruction. The chondrocytes lacked any signs of dystrophic manifestations and were arranged individually or in isogenic groups, which was indicative of the activity of the regenerative processes.

The synovial membranes lacked any signs of pathological changes and hyperplasia; with respect to cellular composition they were represented by synoviocytes and fibroblasts. The total score, based on the system of evaluation of the condition of joint tissues, was equal 22.6, which significantly exceeded the level recorded for the control pathology group and corresponded to the level observed for healthy animals.

### A mixture of the peptides at doses of 300 µg/kg and 600 µg/kg

A study of the morphostructure of the articular cartilages of the rats exposed to the composition according to the invention at doses of 300 and 600 µg/kg revealed cartilage tissues resembling those observed in healthy animals. The values of the articular cartilage thickness and cell density parameters increased significantly in comparison to the control pathology group. The articular cartilages lacked any signs of pronounced pathological changes. The chondrocytes lacked any signs of dystrophic changes and were arranged primarily in isogenic groups, which was indicative of augmentation of the regenerative processes occurring in the cartilaginous tissues.

### Glucosamine sulfate at a dose of 50 mg/kg

Analysis of the slide mounts of the joints of the rats exposed to *glucosamine hydrochloride* at a dose of 50 mg/kg revealed improvement of the morphology in comparison to the control pathology animal group.

### A mixture of the peptides at a dose of 150 µg/kg and glucosamine sulfate at a dose of 50 mg/kg

A study of the morphological structure of the articular cartilages of the rats exposed to the composition according to the invention at a dose of 150 µg/kg in combination with glucosamine sulfate at a dose of 50 mg/kg revealed cartilage tissues resembling those observed in healthy animals. The articular cartilages lacked any signs of pathological changes; the chondrocytes, arranged primarily in isogenic groups, lacked any signs of dystrophic changes. Meanwhile, increase in the articular cartilage thickness and cell density was observed in comparison to the corresponding parameters recorded for animals receiving both the composition of the peptides at doses of 50, 150, 300, and 600 µg /kg and glucosamine sulfate at a dose of 50 mg/kg separately, which was indicative of a synergistic effect associated with the combined use of the peptide composition and glucosamine sulfate.

**Table 2**

| Morphometric parameters of the articular cartilages of the rats receiving the composition according to the invention | | | | |
|---|---|---|---|---|
| Experimental conditions | Dose of the preparation | Cartilage thickness, conventional units | Cell density per conventional unit area | Total score according to the cartilage condition evaluation system |
| Control pathology | - | 13.59±0.57 | 21.90±1.25 | 12.9±1.0 |
| Composition Lys-Asp-Glu, Ala-Asp-Glu, Asp-Glu-Gly | 50 µg/kg | 14.16±0.47 | 27.73±1.59 | 18.2±1.4 |
| | 150 µg/kg 300 µg/kg | 16.50±0.95 16.33±1.15 | 28.38±1.85 34.40±0.60 | 22.6±1.8 24.5±0.9 |
| | 600 µg/kg | 16.71±0.58 | 32.43±1.72 | 23.0±0.75 |
| Glucosamine sulfate | 50 mg/kg | 14.10±1.06 | 23.79±1.80 | 16.2±1.3 |
| Composition (150 µg/kg) Lys-Asp-Glu, Ala-Asp-Glu, Asp-Glu-Gly + Glucosamine sulfate (50 mgkg) | | 17.81±0.5 | 42.40±0.64 | 26.71±0.51 |

| | | | | |
|---|---|---|---|---|
| Note: p<0.05 | | | | |

Analysis of the results obtained enables one to draw a conclusion that all types of morphological markers indicative of early stages of deforming osteoarthritis were present in the joints of the rats exposed to dexamethasone: decrease in cell density; dystrophic changes of chondrocytes; changes in synovial membranes; focal lysis of bone tissue and myelofibrosis.

The administration of various doses of the composition according to the invention substantially reduced the severity of the pathological processes occurring in the articular cartilages of the rats with systemic osteoarthritis. Meanwhile, at doses exceeding 50 µg/kg, the composition according to the invention exerted a therapeutic effect resulting in smoother surfaces of the cartilages that lacked any signs of pulping, normalization of cytoarchitectonics, and more uniform color of the matrix with minimal manifestations of destruction. The administration of the composition at doses of 150, 300, and 600 µg/kg was associated with complete absence of any sings of destruction and lack of dystrophic changes in the chondrocytes.

Therefore, application of the peptide composition in animals with experimental arthritis leads to normalization of the morphostructure of the articular tissues. All this is indicative of reduction in manifestation of the destructive processes occurring in the tissues of the articular cartilages and increase in their regenerative capabilities. Analysis of the data obtained enables one to draw a conclusion that according to the results of the clinical observations and biochemical studies the composition according to the invention exerts a positive effect on the progression of the experimental arthritis in rats receiving daily doses of the investigated compound ranging from 50 to 600 µg/kg and is, consequently, characterized by chondroprotective properties. Meanwhile, the combined use of the peptide composition and glucosamine sulfate is associated with a synergistic effect.

The effect of the composition according to the invention on the intensity of apoptosis of the chondrocytes found in the articular cartilages of rats with systemic steroid arthritis was also investigated. Identification of the apoptosed cells in the articular cartilage tissues was carried out using immunohistochemical techniques on semithin paraffin sections by a TUNEL reaction utilizing In Situ Cell Death Detection Kits manufactured by Roche (Germany). High sensitivity and selectivity represent the primary advantages of this method enabling one to accurately identify the cells at the early stages of cell death.

The results of the immunohistochemical investigation of the articular cartilage structure revealed decrease in the number of chondrocytes undergoing apoptosis (chondroptosis), particularly its final stages, which was indicative of the fact that the composition according to the invention is characterized by antiapoptotic effect, with evident synergistic effect associated with the combined use of the peptide composition and glucosamine sulfate.

### III. Studies of the ultrastructure of the cartilaginous tissue of rats with experimental arthritis

### A mixture of the peptides at a dose of 50 µg/kg

Electron microscopic study of the structure of the articular cartilages of the animals exposed to the influence of the composition according to the invention at a dose of 50 µg/kg revealed the following ultrastructural features: reduced manifestations of the degenerative-dystrophic lesions of the cartilaginous tissues and increased metabolic activity of the chondrocytes.

The surfaces of the cartilages were found to be smooth, covered with dense collagen-containing acellular layers. The chondrocytes identified were characterized by increased heterochromatization of their cell nuclei, which was indicative of decreased intensity of biosynthetic processes. The ultrastructure of the animal cartilages was not characterized by the presence of chondrocytes in a state of necrobiosis or cell death, i.e. with signs of classical apoptosis and chondroptosis. All of the mentioned above was indicative of a positive effect exerted by the composition according to the invention administered at a dose of 50 µg/kg on the ultrastructure of the articular cartilages of the animals belonging to the mentioned group, which was primarily manifested in the enhancement of the metabolic and biosynthetic processes.

### A mixture of the peptides at a dose of 150 µg/kg

The results of a study of the ultrastructure of the articular cartilages of rats exposed to the influence of the composition at a dose of 150 µg/kg were indicative of a significant increase in the metabolic activity of the cartilage cells and reduction in the manifestation of the degenerative-dystrophic processes in comparison with the control pathology group. The surface zones of the cartilages contained matrix characterized by uniform electron density and highly ordered fibrous structure housing oblong immature chondrocytes. High degree of vacuolization of the cytoplasm was a distinctive feature of these cells.

### A mixture of the peptides at doses of 300 and 600 µg/kg

Electron-microscopic study of the structure of the articular cartilages of animals exposed to the influence of the composition administered at doses of 300 and 600 µg/kg revealed significantly reduced manifestations of the degenerative-dystrophic processes in comparison with the control pathology group. The surface areas of the cartilages were characterized by the ultrastructure similar to the ultrastructure of the cartilages observed in healthy animals. The surfaces of the cartilages were found to be smooth, formed by acellular collagen plate. The matrix was characterized by uniform electron density and highly ordered fibrous structure housing oblong immature chondrocytes.

### Glucosamine sulfate at a dose of 50 mg/kg

A study of the influence of glucosamine sulfate administered at a dose of 50 mg/kg on the ultrastructure of the cartilages of rats with systemic arthritis revealed normalization of the ultrastructure of the cartilages accompanied by moderate degenerative-dystrophic manifestations in chondrocytes. It should be emphasized that the majority of cells belonging to this group were characterized by the presence of signs of vacuolar dystrophy of varying severity, which was indicative of a certain degree of activity of the pathological processes that caused dystrophic transformations within the specified cells.

### A mixture of the peptides at a dose of 150 µg/kg and glucosamine sulfate at a dose of 50 mg/kg

The surface zones of the cartilages were characterized by the ultrastructure analogous to the ultrastructure of the cartilages observed in healthy animals.

Analysis of the results obtained enables one to conclude that the ultrastructural organization of the cartilaginous tissues of the rats exposed to dexamethasone administered on three occasions was characterized by all types of morphological manifestations indicative of early stages of osteoarthritis, which primarily included abnormalities in the biosynthetic activity of chondrocytes, development of degenerative-dystrophic changes in the specified cells, increase in cell death by apoptosis (chondroptosis), and destruction of the cartilaginous matrices.

Administration of the composition according to the invention to animals as a chondroprotective agent even at a minimum dose (50 µg/kg) resulted in positive shifts, which manifested in the form of augmentation of the biosynthetic processes occurring within the specified cells. The latter was primarily evidenced by increase in the content of membrane structures and Golgi complexes. Increase in the dose up to 150-600 µg/kg was associated with increase in the magnitude of the chondroprotective activity. The strongest chondroprotective effect was associated with the combined use of the composition of the peptides and glucosamine sulfate. This was primarily manifested in the disappearance of chondrocytes at various stages of cell death from the slide mounts.

Example 3. Administration of the peptide composition and the peptide composition in combination with glucosamine sulfate and/or chondroitin sulfate to patients with degenerative-dystrophic diseases of the joints and the spine.

The studies were carried out in a group patients aged 40 - 65 years.

The first group (50 patients) was characterized by stiffness in the joints, as well as puffiness or swelling in the joints, which was manifested in the appearance of pain of varying intensity and duration.

The second group (30 patients) was characterized by the degenerative process occurring in the intervertebral joints and intervertebral discs accompanied by deformation of the intervertebral discs and formation of osteophytes, which was manifested in the appearance of pain of varying intensity and duration.

A total of 15 patients in the first group were exposed to the peptide composition administered at a dose of 150 µg; 7 patients received glucosamine sulfate at a dose of 500 mg; 10 patients were treated with the composition of the peptides administered at a dose of 150 µg, glucosamine sulfate at a dose of 500 mg, and chondroitin sulfate at a dose of 50 mg; and 18 patients received the composition of the peptides administered at a dose of 150 µg and glucosamine sulfate at a dose of 500 mg in the form of tablets twice a day over the first three weeks and then once daily for up to 6 months. A sustained therapeutic effect associated with administration of glucosamine sulfate was achieved after at least 6 months of therapy. A sustained therapeutic effect associated with administration of the composition of the peptides was achieved after 5-6 months of therapy, while a sustained therapeutic effect associated with administration of the composition of the peptides in combination with glucosamine sulfate or glucosamine sulfate and chondroitin sulfate was achieved after 4 months of therapy.

A total of 10 patients in the second group received the peptide composition at a dose of 300 µg; 9 patients received glucosamine sulfate at a dose of 50 mg; and 11 patients received the composition of the peptides at a dose of 300 µg and glucosamine sulfate at a dose of 50 mg administered intramuscularly on a daily basis. The treatment course comprised 30 injections. A therapeutic effect associated with administration of glucosamine sulfate was achieved after at least 1 month of therapy. A sustained therapeutic effect associated with administration of the composition of the peptides was achieved after 3-4 weeks of therapy, while a sustained therapeutic effect associated with administration of the composition of the peptides in combination with glucosamine sulfate was achieved after 2-3 weeks of therapy.

Therefore, the treatment of the rats with experimental osteoarthritis revealed that the composition of the peptides and the composition of the peptides combined with glucosamine sulfate and/or chondroitin sulfate positively changed the dynamics of the clinical and biochemical parameters, contributed to normalization of the morphostructure of the articular tissues (including cytoarchitectonics of the cartilaginous tissue accompanied by decrease in the number of chondrocytes undergoing apoptosis, especially its final stages), exerted a positive effect on the metabolic processes occurring in the chondrocytes (activation of the synthetic processes and normalization of the biopolymer composition of the cartilaginous matrix), as well as exerted a therapeutic effect in patients with degenerative-dystrophic diseases of the joints and the spine, which was accompanied by a pronounced analgesic effect. Application of the proposed invention enables one to improve the efficacy of treatment of the joint disease. The treatment leads to remission of the disease, gradual decrease in joint pain, decrease in stiffness, and regeneration of cartilaginous tissue. Application of the agents according to the invention in the early stages of diseases of the joints enables one to prevent the development of the pathological processes.

### Example 4. Manufacturing of various dosage forms of the composition.

### Solid forms (tablets, including lozenges and capsules):

Solid forms are manufactured using conventional methods (Chueshov V.I. et al. Industrial Technology of Medicinal Products, vol. 2, MTK-Kniga, Publishing House of the National University of Pharmacy, 2002, pp. 400-413).

In order to manufacture capsules, the relevant ingredients are mixed with allowance for the composition of a single capsule: peptide mixture in powder form, e.g. at a ratio of 1:1:1 - 150 µg; glucosamine (or its salts) - 750 mg; excipients: 5 mg of magnesium stearate, 1 mg of manganese sulfate, and 10 mg of stearic acid.

The mass for encapsulation is prepared as follows: powders of a mixture of peptides and/or glucosamine (or its salts) and/or chondroitin (or its salts), lactose, calcium or magnesium stearate, or any other pharmaceutically acceptable carriers are sieved and weighed. The powders are then mixed at certain ratios of ingredients by weight according to the technical regulations provided by the manufacturing company. The resulting intermediate powder mixture is moistened with 70% ethanol and the moist intermediate mixture is passed through a sieve with a diameter of 3 mm to obtain wet granules (granulate). The granules are dried, dusted, and placed into gelatin capsules.

Tablets are manufactured using conventional methods (Chueshov V.I. et al. Industrial Technology of Medicinal Products, vol. 2, MTK-Kniga, Publishing House of the National University of Pharmacy, 2002, pp. 335-368).

In order to manufacture tablets, the relevant ingredients are mixed with allowance for the composition of a single tablet: peptide mixture (in powder form, e.g. at a ratio of 1:2:1) - 150 µg; chondroitin (and or its salts) - 200 mg; excipients: 4.8 mg of calcium stearate, 12.39 mg of crospovidone, 107.498 mg of magnesium carbonate, 9.312 mg of povidone, and 96 mg of microcrystalline cellulose).

Manufacturing of tablets comprises the following steps: crushing → sieving → (optional wet granulation) → mixing of powders → hydration → granulation → drying → dusting → molding.

Injection solutions are manufactured using conventional methods (Chueshov V.I. et al. Industrial Technology of Medicinal Products, vol. 2, MTK-Kniga, Publishing House of the National University of Pharmacy, 2002, pp. 510-543).

In order to manufacture solution for intravenous and intramuscular administration, the relevant ingredients are mixed with allowance for the contents of a single ampoule (2 ml): a mixture of the peptides in powder form, e.g. at a ratio of 1:3:1 - 100 µg; chondroitin (or its pharmaceutically acceptable salts) - 100 mg; glucosamine (or its pharmaceutically acceptable salts) - 100 mg; water for injection - up to 2 ml.

Or in order to manufacture solution for intravenous and intramuscular administration, the ingredients are mixed with allowance for the contents of a single ampoule (2 ml): a mixture of the peptides in powder form, e.g. at a ratio of 1:2:1 - 150 µg; water for injection - up to 2 ml.

The solution obtained is clear, colorless or slightly yellowish, odorless.

### Nasal dosage form:

Nasal drops - 5 ml (the content of the peptide mixture - 75 µg, glucosamine sulfate - 800 mg).

### Aerosol form:

Aerosol form - 100 ml (the content of the peptide mixture - 1.2 mg, chondroitin sulfate - 2.5 g, glucosamine sulfate - 3.5 g). The preparation is applied topically. The protector cap is removed from the dispenser prior to application; the spray can should be kept vertically when used with the dispenser facing upward; one must not use the spray can when it is inverted. Once the preparation is dispensed, the protective cap is placed back onto the dispenser.

## Claims

1. A pharmaceutical composition comprising peptides Lys-Asp-Glu, Ala-Asp-Glu, Asp-Glu-Gly, chondroitin or its salts, and a pharmaceutically acceptable carrier.

2. The pharmaceutical composition according to claim **1,** manufactured in the form of a tablet, nasal spray, sublingual aerosol, capsule, powder, solution, lyophilisate for preparation of solution, gel, or drops for oral administration.

3. The pharmaceutical composition according to claim **2,** wherein the tablets are sublingual, sustained-release and film-coated.

4. The pharmaceutical composition according to any one of claims **1** to **3,** comprising a weight ratio of peptides Lys-Asp-Glu, Ala-Asp-Glu and Asp-Glu-Gly of 0.01-1.00 %.

5. A pharmaceutical composition comprising peptides Lys-Asp-Glu, Ala-Asp-Glu, Asp-Glu-Gly, glucosamine or its salts and a pharmaceutical acceptable carrier.

6. The pharmaceutical composition according to claim **5,** manufactured in the form of a tablet, nasal spray, sublingual aerosol, capsule, powder, solution, lyophilisate for preparation of solution, gel, or drops for oral administration.

7. The pharmaceutical composition according to claim **6,** wherein the tablets are sublingual, sustained-release and film-coated.

8. The pharmaceutical composition according to any one of claims **5** to **7,** comprising a weight ratio of peptides Lys-Asp-Glu, Ala-Asp-Glu and Asp-Glu-Gly of 0.01-1.00 %.

9. A pharmaceutical composition, comprising peptides Lys-Asp-Glu, Ala-Asp-Glu, Asp-Glu-Gly, chondroitin or its salts, glucosamine or its salts and a pharmaceutically acceptable carrier.

10. The pharmaceutical composition according to claim **9,** manufactured in the form of a tablet, nasal spray, sublingual aerosol, capsule, powder, solution, lyophilisate for preparation of solution, gel, or drops for oral administration.

11. The pharmaceutical composition according to claim **10,** wherein the tablets are sublingual, sustained-release and film-coated.

12. The pharmaceutical composition according to any one of claims **9** to **11,** comprising a weight ratio of peptides Lys-Asp-Glu, Ala-Asp-Glu and Asp-Glu-Gly of 0.01-1.00 %.

13. The pharmaceutical composition according to any one of claims **1** to **4,** for use in the prevention and treatment of diseases of the joints and the spine.

14. The pharmaceutical composition according to any one of claims **5** to **8,** for use in the prevention and treatment of diseases of the joints and the spine.

15. The pharmaceutical composition according to any one of claims **9** to **12,** for use in the prevention and treatment of diseases of the joints and the spine.

## Patentansprüche

1. Pharmazeutische Zusammensetzung, umfassend die Peptide Lys-Asp-Glu, Ala-Asp-Glu, Asp-Glu-Gly, Chondroitin oder seine Salze und einen pharmazeutisch annehmbaren Trägerstoff.

2. Pharmazeutische Zusammensetzung nach Anspruch 1, hergestellt in Form einer Tablette, eines Nasensprays, eines sublingualen Aerosols, einer Kapsel, eines Pulvers, einer Lösung, eines Lyophilisats zur Herstellung einer Lösung, eines Gels oder Tropfen zur oralen Verabreichung.

3. Pharmazeutische Zusammensetzung nach Anspruch **2,** wobei die Tabletten sublingual, zur anhaltenden Freisetzung und filmbeschichtet sind.

4. Pharmazeutische Zusammensetzung nach einem der Ansprüche **1** bis **3,** umfassend ein Gewichtsverhältnis der Peptide Lys-Asp-Glu, Ala-Asp-Glu und Asp-Glu-Gly von 0,01 - 1,00 %.

5. Pharmazeutische Zusammensetzung, umfassend die Peptide Lys-Asp-Glu, Ala-Asp-Glu, Asp-Glu-Gly, Glucosamin oder seine Salze und einen pharmazeutisch annehmbaren Trägerstoff.

6. Pharmazeutische Zusammensetzung nach Anspruch **5,** hergestellt in Form einer Tablette, eines Nasensprays, eines sublingualen Aerosols, einer Kapsel, eines Pulvers, einer Lösung, eines Lyophilisats zur Herstellung einer Lösung, eines Gels oder Tropfen zur oralen Verabreichung.

7. Pharmazeutische Zusammensetzung nach Anspruch **6,** wobei die Tabletten sublingual, zur anhaltenden Freisetzung und filmbeschichtet sind.

8. Pharmazeutische Zusammensetzung nach einem der Ansprüche **5** bis **7,** umfassend ein Gewichtsverhältnis der Peptide Lys-Asp-Glu, Ala-Asp-Glu und Asp-Glu-Gly von 0,01 - 1,00 %.

9. Pharmazeutische Zusammensetzung, umfassend die Peptide Lys-Asp-Glu, Ala-Asp-Glu, Asp-Glu-Gly, Chondroitin oder seine Salze, Glucosamin oder seine Salze und einen pharmazeutisch annehmbaren Trägerstoff.

10. Pharmazeutische Zusammensetzung nach Anspruch **9,** hergestellt in Form einer Tablette, eines Nasensprays, eines sublingualen Aerosols, einer Kapsel, eines Pulvers, einer Lösung, eines Lyophilisats zur Herstellung einer Lösung, eines Gels oder Tropfen zur oralen Verabreichung.

11. Pharmazeutische Zusammensetzung nach Anspruch **10,** wobei die Tabletten sublingual, zur anhaltenden Freisetzung und filmbeschichtet sind.

12. Pharmazeutische Zusammensetzung nach einem der Ansprüche **9** bis **11,** umfassend ein Gewichtsverhältnis der Peptide Lys-Asp-Glu, Ala-Asp-Glu und Asp-Glu-Gly von 0,01 - 1,00 %.

13. Pharmazeutische Zusammensetzung nach einem der Ansprüche **1** bis **4** zur Verwendung bei der Vorbeugung und Behandlung von Erkrankungen der Gelenke und der Wirbelsäule.

14. Pharmazeutische Zusammensetzung nach einem der Ansprüche **5** bis **8** zur Verwendung bei der Vorbeugung und Behandlung von Erkrankungen der Gelenke und der Wirbelsäule.

15. Pharmazeutische Zusammensetzung nach einem der Ansprüche **9** bis **12** zur Verwendung bei der Vorbeugung und Behandlung von Erkrankungen der Gelenke und der Wirbelsäule.

## Revendications

1. Une composition pharmaceutique comprenant les peptides Lys-Asp-Glu, Ala-Asp-Glu, Asp-Glu-Gly, de la chondroïtine ou ses sels, et un excipient pharmaceutiquement acceptable

2. La composition pharmaceutique selon la revendication **1,** manufacturée sous la forme d'un comprimé, d'un spray nasal, d'un aérosol sublingual, d'une capsule, d'une poudre, d'une solution, lyophilisée pour la préparation de solution, d'un gel, ou de gouttes pour une administration orale.

3. La composition pharmaceutique selon la revendication **2,** dans laquelle les comprimés sont sublinguaux, à libération prolongée et pelliculés.

4. La composition pharmaceutique selon l'une selon l'une quelconque des revendications **1** à **3,** comprenant un rapport pondéral de peptides Lys-Asp-Glu, Ala-Asp-Glu and Asp-Glu-Gly de 0,01-1,00%.

5. Une composition pharmaceutique comprenant les peptides Lys-Asp-Glu, Ala-Asp-Glu, Asp-Glu-Gly, de la glucosamine ou ses sels, et un excipient pharmaceutiquement acceptable

6. La composition pharmaceutique selon la revendication **5,** manufacturée sous la forme d'un comprimé, d'un spray nasal, d'un aérosol sublingual, d'une capsule, d'une poudre, d'une solution, lyophilisée pour la préparation d'une solution, d'un gel, ou de gouttes pour une administration orale.

7. La composition pharmaceutique selon la revendication **6,** dans laquelle les comprimés sont sublinguaux, à libération prolongée et pelliculés.

8. La composition pharmaceutique selon l'une selon l'une quelconque des revendications **5** à **7,** comprenant un rapport pondéral de peptides Lys-Asp-Glu, Ala-Asp-Glu and Asp-Glu-Gly de 0,01-1,00%.

9. Une composition pharmaceutique comprenant les peptides Lys-Asp-Glu, Ala-Asp-Glu, Asp-Glu-Gly, de la chondroïtine ou ses sels, de la glucosamine ou ses sels et un excipient pharmaceutiquement acceptable

10. La composition pharmaceutique selon la revendication **9,** manufacturée sous la forme d'un comprimé, d'un spray nasal, d'un aérosol sublingual, d'une capsule, d'une poudre, d'une solution, lyophilisée pour la préparation d'une solution, d'un gel, ou de gouttes pour une administration orale.

11. La composition pharmaceutique selon la revendication **10,** dans laquelle les comprimés sont sublinguaux, à libération prolongée et pelliculés.

12. La composition pharmaceutique selon l'une selon l'une quelconque des revendications **9** à **11,** comprenant un rapport pondéral de peptides Lys-Asp-Glu, Ala-Asp-Glu and Asp-Glu-Gly de 0,01-1,00%.

13. La composition pharmaceutique selon l'une selon l'une quelconque des revendications **1** à **4,** pour son utilisation dans la prévention et le traitement des maladies des articulations et de la colonne vertébrale.

14. La composition pharmaceutique selon l'une selon l'une quelconque des revendications **5** à **8,** pour son utilisation dans la prévention et le traitement des maladies des articulations et de la colonne vertébrale.

15. La composition pharmaceutique selon l'une selon l'une quelconque des revendications **9** à **12,** pour son utilisation dans la prévention et le traitement des maladies des articulations et de la colonne vertébrale.
